# FASCICULE DE BREVET EUROPEEN

(11) **EP 3 016 506 B1**
(45) Date de publication et mention de la délivrance du brevet: **12.09.2018**
(21) Numéro de dépôt: 14752936.6
(22) Date de dépôt: 30.06.2014
(51) Int. Cl.: A01H 5/00, C07K 14/415, C12N 15/82

(54) **AUGMENTATION DE LA RECOMBINAISON MÉIOTIQUE CHEZ LES PLANTES PAR INHIBITION DE LA PROTÉINE FIDG**
ERHÖHUNG DER MEIOTISCHEN REKOMBINATION IN PFLANZEN DURCH HEMMUNG DES FIDG-PROTEINS
INCREASED MEIOTIC RECOMBINATION IN PLANTS BY INHIBITION OF THE FIDG PROTEIN

(30) Priorité: 03.07.2013 FR 1356522
(43) Date de publication de la demande: 11.05.2016
(73) Titulaire: INSTITUT NATIONAL DE LA RECHERCHE AGRONOMIQUE, 75007 Paris (FR)
(72) Inventeur: MERCIER, Raphaël, F-78340 Les Clayes sous Bois (FR); GIRARD, Chloé, 92330 Sceaux (FR); CRISMANI, Wayne, Victoria 3058 (AU)
(74) Mandataire: Gevers & Orès
(86) Numéro de dépôt international: PCT/IB2014/062724
(87) Numéro de publication internationale: WO 2015/001467

(56) Documents cités:
- WO-A1-2013/038376
- CRISMANI WAYNE ET AL: "What limits meiotic crossovers?", CELL CYCLE, vol. 11, no. 19, octobre 2012 (2012-10), pages 3527-3528, XP002721863,
- COX GREGORY A ET AL: "The mouse fidgetin gene defines a new role for AAA family proteins in mammalian development", NATURE GENETICS, vol. 26, no. 2, octobre 2000 (2000-10), pages 198-202, XP002721864, ISSN: 1061-4036 cité dans la demande
- L'HOTE DAVID ET AL: "Fidgetin-Like1 Is a Strong Candidate for a Dynamic Impairment of Male Meiosis Leading to Reduced Testis Weight in Mice", PLOS ONE, vol. 6, no. 11, novembre 2011 (2011-11), XP002721865, ISSN: 1932-6203 cité dans la demande
- YUAN J ET AL: "FIGNL1-containing protein complex is required for efficient homologous recombination repair", PROCEEDINGS OF THE NATIONAL ACADEMY OF SCIENCES OF THE UNITED STATES OF AMERICA 20130625 NATIONAL ACADEMY OF SCIENCES USA, vol. 110, no. 26, 25 juin 2013 (2013-06-25), pages 10640-10645, XP002721871, ISSN: 0027-8424

### SEQUENCE LISTING

<110> INSTITUT NATIONAL DE LA RECHERCHE AGRONOMIQUE MERCIER, Raphaël GIRARD, Chloé CRISMANI, Wayne
<120> AUGMENTATION DE LA RECOMBINAISON MÉIOTIQUE CHEZ LES PLANTES PAR INHIBITION DE LA PROTÉINE FIDG
<130> MJP-11-F539-164PCT
<150> FR1356522
   <151> 2013-07-03
<160> 2
<170> PatentIn version 3.5
<210> 1
   <211> 672
   <212> PRT
   <213> Arabidopsis thaliana
<220>
   <221> DOMAIN
   <222> (268)..(346)
   <223> Domaine de liaison à RAD51
<220>
   <221> DOMAIN
   <222> (431)..(561)
   <223> Domaine AAA
<220>
   <221> DOMAIN
   <222> (623)..(672)
   <223> Domaine VPS4
<400> 1
   Met Cys Gly Ser Val Val Ser Asp Ser Glu Arg Glu Phe Glu Phe His
   1               5                   10                  15
   Phe Trp Gly Phe Gly His Leu Gly Lys Glu Glu Glu Arg Ile Met Ala
               20                  25                  30
   Gly Lys Arg Ser Ser Pro Phe Ser Ser Pro Leu Leu Arg Pro Pro Pro
           35                  40                  45
   Glu Phe Leu Asn Val Lys Glu Glu Gly Glu Thr Glu Thr Pro Cys Trp
       50                  55                  60
   Arg Lys Glu Val Asp Glu Asn Leu Lys Arg Leu Gln Ser Leu Leu Phe
   65                  70                  75                  80
   Gly Ala Asp Lys Phe Leu Glu Lys Ser Asp Phe Ser Ser Ala Gln Ile
                   85                  90                  95
   Leu Gly Leu Arg Leu Leu Gly Phe Leu Asp Ser Arg Ser Val Thr Asp
               100                 105                 110
   Ala Asp Arg Asp Phe Ile Gly Pro Ile Arg Arg Glu Val Ala Ser Lys
           115                 120                 125
   Ile Asp Leu Ala Leu Glu Gly Leu Val Ser Asp Ser Asp Arg Lys Ala
       130                 135                 140
   Phe Glu Leu Ala Asn Thr Ala Pro Gly Ala Ile Phe Gly Ser Lys Gly
   145                 150                 155                 160
   Gly Phe Asp Val Glu Lys Ile Lys Gln Ser Lys Tyr Phe Gly Phe His
                   165                 170                 175
   Val Ser Gln Ser Asn Gly Lys Gly Val Lys Glu Met Leu Ile Pro Lys
               180                 185                 190
   Ala Pro Lys Ser Met Met Gln Ala Lys Leu Thr Ser Leu Tyr Gly Asn
           195                 200                 205
   Ser Ile Gly Lys Pro Asp Asn Gln Arg Lys Thr Ser Val Asn Asn Gln
       210                 215                 220
   Asp Arg Ala Ser Asp Glu Cys Val Ile Val Glu Arg Ser His Gly Phe
   225                 230                 235                 240
   Gly Phe Gly Thr Lys Arg Pro His Ala Glu Thr Ser Ser Leu Ala Asn
                   245                 250                 255
   Asp Gly Glu Val Lys Glu Asp Gly Ala Pro Asn Gly Phe Val Ser Ala
               260                 265                 270
   Lys Ile Lys Leu Glu Met Asp Val Arg Gln Lys Arg Gly Ser Thr Glu
           275                 280                 285
   Ser Pro Ser Ser Cys Leu Ser Pro Gln Ser Asp Lys Asn Ala Leu Gly
       290                 295                 300
   Arg Gly Tyr Gly Ser Arg Ser Gly Gly Leu Arg Arg Gly Tyr Arg Ser
   305                 310                 315                 320
   Asn Phe Val Pro Pro Val Lys Thr Asn Gly Asn Asn Val Gly Asn Leu
                   325                 330                 335
   Thr Ser Arg Ile Gly Gly Lys Thr Asp Asp Ala Leu Asp Asp Ser Thr
               340                 345                 350
   Arg Thr Cys Leu Glu Met Leu Cys Gly Pro Asp Gly Glu Leu Pro Glu
           355                 360                 365
   Lys Leu Arg Asn Leu Glu Pro Arg Leu Ile Glu His Val Ser Asn Glu
       370                 375                 380
   Ile Met Asp Arg Asp Pro Asn Val Arg Trp Asp Asp Ile Ala Gly Leu
   385                 390                 395                 400
   Glu His Ala Lys Lys Cys Val Thr Glu Met Val Ile Trp Pro Leu Leu
                   405                 410                 415
   Arg Pro Asp Ile Phe Lys Gly Cys Arg Ser Pro Gly Lys Gly Leu Leu
               420                 425                 430
   Leu Phe Gly Pro Pro Gly Thr Gly Lys Thr Met Ile Gly Lys Ala Ile
           435                 440                 445
   Ala Gly Glu Ala Lys Ala Thr Phe Phe Tyr Ile Ser Ala Ser Ser Leu
       450                 455                 460
   Thr Ser Lys Trp Ile Gly Glu Gly Glu Lys Leu Val Arg Ala Leu Phe
   465                 470                 475                 480
   Gly Val Ala Ser Cys Arg Gln Pro Ala Val Ile Phe Val Asp Glu Ile
                   485                 490                 495
   Asp Ser Leu Leu Ser Gln Arg Lys Ser Asp Gly Glu His Glu Ser Ser
               500                 505                 510
   Arg Arg Leu Lys Thr Gln Phe Leu Ile Glu Met Glu Gly Phe Asp Ser
           515                 520                 525
   Gly Ser Glu Gln Ile Leu Leu Ile Gly Ala Thr Asn Arg Pro Gln Glu
       530                 535                 540
   Leu Asp Glu Ala Ala Arg Arg Arg Leu Thr Lys Arg Leu Tyr Ile Pro
   545                 550                 555                 560
   Leu Pro Ser Ser Glu Ala Arg Ala Trp Ile Ile Gln Asn Leu Leu Lys
                   565                 570                 575
   Lys Asp Gly Leu Phe Thr Leu Ser Asp Asp Asp Met Asn Ile Ile Cys
               580                 585                 590
   Asn Leu Thr Glu Gly Tyr Ser Gly Ser Asp Met Lys Asn Leu Val Lys
   595 600 605
   Asp Ala Thr Met Gly Pro Leu Arg Glu Ala Leu Lys Arg Gly Ile Asp
   610 615 620
   Ile Thr Asn Leu Thr Lys Asp Asp Met Arg Leu Val Thr Leu Gln Asp
   625 630 635 640
   Phe Lys Asp Ala Leu Gln Glu Val Arg Pro Ser Val Ser Gln Asn Glu
   645 650 655
   Leu Gly Ile Tyr Glu Asn Trp Asn Asn Gln Phe Gly Ser Leu Ser Leu
   660 665 670
<210> 2
   <211> 1390
   <212> PRT
   <213> Arabidopsis thaliana
<220>
   <221> DOMAIN
   <222> (129)..(272)
   <223> Domaine hélicase DEXDc
<220>
   <221> DOMAIN
   <222> (445)..(570)
   <223> Domaine hélicase HELICc
<400> 2
   Met Gly Ser Arg Val Pro Ile Glu Thr Ile Glu Glu Asp Gly Glu Phe
   1 5 10 15
   Asp Trp Glu Ala Ala Val Lys Glu Ile Asp Leu Ala Cys Leu Lys Thr
   20 25 30
   Thr Asn Ala Ser Ser Ser Ser Ser Ser His Phe Thr Pro Leu Ala Asn
   35 40 45
   Pro Pro Ile Thr Ala Asn Leu Thr Lys Pro Pro Ala Lys Arg Gln Ser
   50 55 60
   Thr Leu Asp Lys Phe Ile Gly Arg Thr Glu His Lys Pro Glu Asn His
   65 70 75 80
   Gln Val Val Ser Glu Cys Gly Val Asn Asp Asn Asp Asn Ser Pro Leu
   85 90 95
   Val Gly Ile Asp Pro Glu Ala Ala Lys Thr Trp Ile Tyr Pro Gly Phe
               100                 105                 110
   Val Pro Leu Arg Asp Tyr Gln Phe Ala Ile Thr Lys Thr Ala Leu Phe
           115                 120                 125
   Ser Asn Thr Leu Val Ala Leu Pro Thr Gly Leu Gly Lys Thr Leu Ile
       130                 135                 140
   Ala Ala Val Val Met Tyr Asn Tyr Phe Arg Trp Phe Pro Gln Gly Lys
   145                 150                 155                 160
   Ile Val Phe Ala Ala Pro Ser Arg Pro Leu Val Met Gln Gln Ile Glu
                   165                 170                 175
   Ala Cys His Asn Ile Val Gly Ile Pro Gln Glu Trp Thr Ile Asp Leu
               180                 185                 190
   Thr Gly Gln Thr Cys Pro Ser Lys Arg Ala Phe Leu Trp Lys Ser Lys
           195                 200                 205
   Arg Val Phe Phe Val Thr Pro Gln Val Leu Glu Lys Asp Ile Gln Ser
       210                 215                 220
   Gly Thr Cys Leu Thr Asn Tyr Leu Val Cys Leu Val Ile Asp Glu Ala
   225                 230                 235                 240
   His Arg Ala Leu Gly Asn Tyr Ser Tyr Cys Val Val Val Arg Glu Leu
                   245                 250                 255
   Met Ala Val Pro Ile Gln Leu Arg Ile Leu Ala Leu Thr Ala Thr Pro
               260                 265                 270
   Gly Ser Lys Thr Gln Ala Ile Gln Gly Ile Ile Asp Asn Leu Gln Ile
           275                 280                 285
   Ser Thr Leu Glu Tyr Arg Asn Glu Ser Asp His Asp Val Cys Pro Tyr
       290                 295                 300
   Val His Asp Arg Lys Leu Glu Val Ile Glu Val Pro Leu Gly Gln Asp
   305                 310                 315                 320
   Ala Asp Asp Val Ser Lys Arg Leu Phe His Val Ile Arg Pro Tyr Ala
                   325                 330                 335
   Val Arg Leu Lys Asn Phe Gly Val Asn Leu Asn Arg Asp Ile Gln Thr
               340                 345                 350
   Leu Ser Pro His Glu Val Leu Met Ala Arg Asp Lys Phe Arg Gln Ala
           355                 360                 365
   Pro Leu Pro Gly Leu Pro His Val Asn His Gly Asp Val Glu Ser Cys
       370                 375                 380
   Phe Ala Ala Leu Ile Thr Leu Tyr His Ile Arg Lys Leu Leu Ser Ser
   385                 390                 395                 400
   His Gly Ile Arg Pro Ala Tyr Glu Met Leu Glu Glu Lys Leu Lys Glu
                   405                 410                 415
   Gly Pro Phe Ala Arg Leu Met Ser Lys Asn Glu Asp Ile Arg Met Thr
               420                 425                 430
   Lys Leu Leu Met Gln Gln Arg Leu Ser His Gly Ala Pro Ser Pro Lys
           435                 440                 445
   Leu Ser Lys Met Leu Glu Ile Leu Val Asp His Phe Lys Val Lys Asp
       450                 455                 460
   Pro Lys Thr Ser Arg Val Ile Ile Phe Ser Asn Phe Arg Gly Ser Val
   465                 470                 475                 480
   Arg Asp Ile Met Asn Ala Leu Ser Asn Ile Gly Asp Met Val Lys Ala
                   485                 490                 495
   Thr Glu Phe Ile Gly Gln Ser Ser Gly Lys Thr Leu Lys Gly Gln Ser
               500                 505                 510
   Gln Lys Ile Gln Gln Ala Val Leu Glu Lys Phe Arg Ala Gly Gly Phe
           515                 520                 525
   Asn Val Ile Val Ala Thr Ser Ile Gly Glu Glu Gly Leu Asp Ile Met
       530                 535                 540
   Glu Val Asp Leu Val Ile Cys Phe Asp Ala Asn Val Ser Pro Leu Arg
   545                 550                 555                 560
   Met Ile Gln Arg Met Gly Arg Thr Gly Arg Lys Asn Asn Gly Arg Pro
                   565                 570                 575
   Leu Leu Val Leu Ala Cys Glu Gly Ser Glu Lys Asn Ser Tyr Met Arg
               580                 585                 590
   Lys Gln Ala Ser Gly Arg Ala Ile Lys Lys His Met Arg Asn Gly Gly
           595                 600                 605
   Thr Asn Ser Phe Asn Phe His Pro Ser Pro Arg Met Ile Pro His Val
       610                 615                 620
   Tyr Lys Pro Glu Val Gln His Val Glu Phe Ser Ile Lys Gln Phe Val
   625                 630                 635                 640
   Pro Arg Gly Lys Lys Leu Gln Glu Glu Tyr Ala Thr Glu Thr Pro Ala
                   645                 650                 655
   Phe Gln Lys Lys Leu Thr Pro Ala Glu Thr His Met Leu Ala Lys Tyr
               660                 665                 670
   Tyr Asn Asn Pro Asp Glu Glu Lys Leu Arg Val Ser Leu Ile Ala Phe
           675                 680                 685
   Pro His Phe Gln Thr Leu Pro Ser Lys Val His Lys Val Met His Ser
       690                 695                 700
   Arg Gln Thr Gly Met Leu Ile Asp Ala Met Gln His Leu Gln Glu Pro
   705                 710                 715                 720
   Thr Phe Ser Glu Gln Ser Lys Ser Phe Phe Thr Glu Phe Arg Ala Pro
                   725                 730                 735
   Leu Gly Glu Arg Glu Glu Leu Asp Thr Gly Leu Arg Val Thr Asn Asp
               740                 745                 750
   Pro Lys Asp Leu His Ser Val Arg Asp Leu Glu Val Asn Thr Ser Gln
           755                 760                 765
   Arg Lys Ala Lys Gln Val Glu Ser Pro Thr Ser Thr Leu Glu Thr Thr
       770                 775                 780
   Glu Lys Asp Tyr Glu Glu Ser Ser Pro Thr His Arg Tyr Leu Phe Ser
   785                 790                 795                 800
   Ser Glu Cys Ala Ser Val Asp Thr Leu Gly Asn Val Phe Val Met Pro
                   805                 810                 815
   Val Pro Leu Leu Phe Phe Pro Asn Val Leu Glu Ser Asp Asn Thr Pro
               820                 825                 830
   Leu Pro Lys Thr Glu Lys Gln His Ser Cys Arg Asn Thr Ser His Ile
           835                 840                 845
   Asp Leu Val Pro Val Asp Thr Ser Glu Lys His Arg Gln Asp Asn Ile
   850 855 860
   Ser Cys Lys Leu Lys Glu Arg Phe Ser Pro Asp Gly Ala Ser Glu Thr
   865 870 875 880
   Leu Glu Thr His Ser Leu Val Lys Arg Asn Ser Thr Arg Val Gly Glu
   885 890 895
   Asp Asp Val Ala Asn Ser Val Gly Glu Ile Val Leu Ser Ser Asp Glu
   900 905 910
   Asp Asp Cys Glu Gly Leu Glu Leu Ser Pro Arg Leu Thr Asn Phe Ile
   915 920 925
   Lys Ser Gly Ile Val Pro Glu Ser Pro Val Tyr Asp Gln Gly Glu Ala
   930 935 940
   Asn Arg Glu Glu Asp Leu Glu Phe Pro Gln Leu Ser Ser Pro Met Arg
   945 950 955 960
   Phe Ser Asn Glu Leu Ala Gly Glu Ser Ser Phe Pro Glu Arg Lys Val
   965 970 975
   Gln His Lys Cys Asn Asp Tyr Asn Ile Val Ser Thr Thr Thr Glu Leu
   980 985 990
   Arg Thr Pro Gln Lys Glu Val Gly Leu Ala Asn Gly Thr Glu Cys Leu
   995 1000 1005
   Ala Val Ser Pro Ile Pro Glu Asp Trp Arg Thr Pro Leu Ala Asn
   1010 1015 1020
   Leu Thr Asn Thr Asn Ser Ser Ala Arg Lys Asp Trp Arg Val Ser
   1025 1030 1035
   Ser Gly Glu Lys Leu Glu Thr Leu Arg Gln Pro Arg Lys Leu Lys
   1040 1045 1050
   Arg Leu Arg Arg Leu Gly Asp Cys Ser Ser Ala Val Lys Glu Asn
   1055 1060 1065
   Tyr Pro Gly Ile Thr Glu Ala Asp His Ile Arg Ser Arg Ser Arg
   1070 1075 1080
   Gly Lys Lys His Ile Arg Val Lys Trp Lys Ser Ala Glu Ser Trp
   1085 1090 1095
   Phe Thr Leu Ser Ser Val Ala Phe Ala Leu Val Thr Lys Lys Lys
       1100                1105                1110
   Lys Met Ile Met Asp Asp Asp Val Gln Val Phe Ile Asp Glu Glu
       1115                1120                1125
   Ala Glu Val Ser Ser Gly Ala Glu Met Ser Ala Asp Glu Asn Glu
       1130                1135                1140
   Asp Val Thr Gly Asp Ser Phe Glu Asp Ser Phe Ile Asp Asp Gly
       1145                1150                1155
   Thr Met Pro Thr Ala Asn Thr Gln Ala Glu Ser Gly Lys Val Asp
       1160                1165                1170
   Met Met Ala Val Tyr Arg Tyr Ile Gln Pro Lys Ile Ser Phe Phe
       1175                1180                1185
   Tyr Cys Glu Val Asn Glu Leu Ile Lys Asn His Lys Val Ser Phe
       1190                1195                1200
   His Arg Arg Ser Leu Leu Ser Gln Ser Pro Leu Pro Ala Arg Phe
       1205                1210                1215
   Arg Asp Leu Ala Ala Ser Ser Leu Ser Pro Tyr Ser Ala Gly Pro
       1220                1225                1230
   Leu Thr Arg Ile Asn Glu Ser Arg Ser Asp Ser Asp Lys Ser Leu
       1235                1240                1245
   Ser Ser Leu Arg Thr Pro Lys Thr Thr Asn Ser Glu Ser Asn Gln
       1250                1255                1260
   Asp Ala Met Met Ile Gly Asn Leu Ser Val Val Gln Ile Ser Ser
       1265                1270                1275
   Asp Ser Arg Lys Arg Lys Phe Ser Leu Cys Asn Ser Ala Asn Ala
       1280                1285                1290
   Pro Val Ile Asn Leu Glu Ser Lys Phe Ala Ala His Ala Gln Ala
       1295                1300                1305
   Thr Glu Lys Glu Ser His Glu Gly Val Arg Ser Asn Ala Gly Ala
       1310                1315                1320
   Leu Glu Tyr Asn Asp Asp Asp Asp Asp Ala Phe Phe Ala Thr Leu
       1325                1330                1335
   Asp Phe  Asp Ala Met Glu Ala  Gln Ala Thr Leu Leu  Leu Ser Lys
       1340                 1345                 1350
   Gln Arg  Ser Glu Ala Lys Glu  Lys Glu Asp Ala Thr  Val Ile Pro
       1355                 1360                 1365
   Asn Pro  Gly Met Gln Arg Ser  Asp Gly Met Glu Lys  Asp Ala Pro
       1370                 1375                 1380
   Ser Phe  Asp Leu Gly Leu Trp
       1385                 1390

## Description

La présente invention est relative à une méthode pour augmenter la recombinaison méiotique chez les plantes.

La recombinaison méiotique est un échange d'ADN entre chromosomes homologues au cours de la méiose ; elle intervient pendant la prophase de la première division méiotique. Un des produits de la recombinaison est le crossing-over, qui entraine un échange réciproque de continuité entre deux chromatides homologues. Cette prophase (prophase I) comprend 5 stades successifs : leptotène, zygotène, pachytène, diplotène, et diacinèse. Au leptotène les chromosomes s'individualisent, chaque chromosome étant formé de deux chromatides soeurs issues de la duplication intervenue préalablement à la prophase. Durant le zygotène, les chromosomes homologues s'apparient, formant une structure appelée « bivalent » qui contient quatre chromatides, deux chromatides soeurs maternelles et deux chromatides soeurs paternelles, homologues des chromatides maternelles. Au pachytène les chromosomes sont totalement appariés, et des nodules de recombinaison se forment entre les chromatides homologues étroitement reliées entre elles par le complexe synaptonémal (SC); au diplotène, le SC se dissocie progressivement, et les chromosomes homologues commencent à se séparer, mais restent joints au niveau des chiasmas, qui correspondent aux sites de crossing-overs (COs). Les chromosomes se condensent au cours de la diacinèse ; les chiasmas subsistent jusqu'a la métaphase I pendant laquelle ils maintiennent l'appariement des bivalents de part et d'autre de la plaque équatoriale.

La recombinaison méiotique est déclenchée par la formation de cassures double-brin (CDB) dans l'une ou l'autre des chromatides homologues, et résulte de la réparation de ces cassures, utilisant comme matrice une chromatide du chromosome homologue.

La recombinaison méiotique a pour conséquence de provoquer un réassortiment des allèles d'origine paternelle et maternelle dans le génome, ce qui contribue à générer de la diversité génétique. Elle présente donc un intérêt particulier dans les programmes d'amélioration des plantes (WIJNKER & de JONG, Trends in Plant Science, 13, 640-646, 2008, CRISMANI et al, Journal of Experimental Botany, 64, 55-65, 2013). Notamment, un contrôle du taux de recombinaison peut permettre d'augmenter le brassage génétique, et donc les probabilités d'obtenir de nouvelles combinaisons de caractéristiques ; elle peut permettre aussi de faciliter l'introgression de gènes d'intérêt, ainsi que la cartographie génétique et le clonage positionnel de gènes d'intérêt.

Diverses méthodes pour contrôler la recombinaison méiotique ont été proposées, basées sur la sur-expression ou l'extinction de l'un ou l'autre des très nombreux gènes identifiés comme étant impliqués ou potentiellement impliqués dans ce mécanisme. Par exemple, la Demande PCT WO/0208432 propose la surexpression de la protéine RAD51, qui est impliquée dans la recombinaison homologue, pour stimuler la recombinaison méiotique ; la Demande US 2004023388 propose de surexprimer un activateur de la recombinaison méiotique choisi parmi : SPO11, MRE11, RAD50, XRS2/NBS1, DMC1, RAD51, RPA, MSH4, MSHS, MLH1, RAD52, RAD54, TID1, RAD5S, RADS7, RADS9, une résolvase, une protéine liant l'ADN simple brin, une protéine impliquée dans le remodelage de la chromatine, ou une protéine du complexe synaptonémal, pour augmenter la fréquence de recombinaison entre chromatides homologues ; la Demande PCT WO 2004016795 propose d'augmenter la recombinaison entre chromosomes homologues en exprimant une protéine SPO11 fusionnée à un domaine de liaison à l'ADN ; la Demande PCT WO 03104451 propose d'augmenter le potentiel de recombinaison entre des chromosomes homologues par la surexpression d'une protéine (MutS) impliquée dans la réparation des mésappariements.

Cependant, dans la plupart des cas, l'effet de ces gènes candidats sur la formation de COs méiotiques *in planta* n'a pas été confirmé, et il est donc nécessaire d'identifier d'autres gènes intervenant dans ce phénomène.

L'un des facteurs connus pour agir sur le taux de recombinaison méiotique est le phénomène d'interférence: la formation d'un CO à un endroit du chromosome inhibe la formation d'autres COs à proximité. Toutefois, il a été montré qu'il existait en fait 2 voies distinctes de formation des COs méiotiques (HOLLINGSWORTH & BRILL, Genes Dev., 18,117-125, 2004 ; BERCHOWITZ & COPENHAVER, Current genomics, 11, 91-102, 2010). La première génère des COs interférents dénommés COs de type I (COI), et fait intervenir un ensemble de gènes collectivement désignés sous les appellations de gènes *ZMM* (*ZIP1, ZIP2*/*SHOC1, ZIP3, HEI10, ZIP4, MER3, MSH4, MSH5, PTD*) ainsi que les protéines *MLH1* et *MLH3* ; la seconde voie génère des COs non-interférents, dénommés COs de type II (COII), et dépend du gène *MUS81.*

L'utilisation de l'une et/ou l'autre de ces deux voies est variable d'un organisme à l'autre. Chez les végétaux supérieurs, représentés par la plante modèle *Arabidopsis thaliana,* les 2 voies coexistent, celle des COs de type I étant prédominante ; il a été observé que l'inactivation des gènes *AtMSH4, AtMSH5, AtMER3, SHOC1, PTD, AtHEI10* ou *AtZIP4* (induisait jusqu'à 85% de réduction de la fréquence des COs (HIGGINS et al., 2004, précité ; MERCIER et al., 2005, précité ; CHELYSHEVA et al., PLoS Genet. 3, e83, 2007 ; HIGGINS et al., Plant J., 55, 28-39, 2008 ; MACAISNE et al., Current Biology, 18, 1432-1437, 2008; WIJERATNE et al, Molecular Biology of the cell, 17, 1331-43; CHELYSHEVA et al, PLoS Genetics 8, e1002799, 2012). Certains chromosomes homologues ne sont de ce fait plus appariés sous forme de bivalents, mais apparaissent sous forme d'univalents. Cette diminution du nombre de bivalents s'accompagne d'une forte réduction de la fertilité.

Précédemment, les Inventeurs ont identifié un gène, dénommé *FANCM* (pour « *Fanconi Anémia Complementation Group M* »), dont l'inactivation compensait les effets de celle d'*AtMSH5,* de *SHOC1,* ou d'*AtZIP4*, et permettait d'augmenter le nombre de COs méiotiques (Demande PCT WO 2013/038376).

En poursuivant leurs recherches, les Inventeurs ont maintenant identifié un autre gène dont l'inactivation produit des effets similaires à celle de *FANCM.*

Il s'agit du gène « *FIDGETIN* » (*FIDG*) d'*Arabidopsis thaliana (AtFIDG) :* son inactivation compense les effets de celle d'*AtMSH5,* de *SHOC1,* d'*AtZIP4*, ou *d'AtHEI10* et permet d'augmenter le nombre de COs méiotiques et de restaurer la fertilité chez les mutants *zmm Atzip4-*/*-, Atmsh5-*/*-, shoc1-*/*-* et *Athei10-*/*-.* Les Inventeurs ont en outre découvert que les effets de l'inactivation du gène *FIDG* sur l'augmentation du nombre de COs méiotiques se produisaient non seulement chez les mutants *zmm,* mais également chez les plantes de type sauvage possédant des gènes *ZMM* fonctionnels, et que lorsque l'inactivation du gène *FIDG* était combinée à celle du gène *FANCM,* le nombre de COs méiotiques était encore augmenté par rapport à ceux observés lorsque ces gènes étaient inactivés séparément. Par ailleurs, aucune influence de l'inactivation du gène *FIDG* sur le phénotype somatique n'a été observée.

Le premier mutant *fidg* a été décrit dès 1943 (Grüneberg, Journal of Genetics, 45, 22-28, 1943), comme étant associé chez la souris à un nouveau phénotype comprenant des hochements répétés de la tête et des mouvements circulaires de l'animal. COX et al. (Nat Genet, 26, 198-202, 2000) ont identifié le gène *FIDGETIN* dont la mutation était responsable de ce phénotype, et ont également découvert l'existence, chez la souris, de 2 gènes apparentés qui ont été dénommés *FIDGETIN-LIKE 1* et *FIDGETIN-LIKE 2.* Différentes fonctions des protéines FIDG ont été décrites, les impliquant notamment dans la régulation de la prolifération et de la différenciation des ostéoblastes (PARK et al., J Bone Miner Res, 22, 889-96, 2007), la régulation de la spermatogenèse (L'HOTE et al., PLoS One, 6, e27582, 2011), le sectionnement des microtubules (MUKHERJEE et al., Cell Cycle, 11, 2359-66, 2012), et la participation à un complexe, contenant hsRAD51, impliqué dans la réparation de cassures double-brin dans des cellules somatiques (YUAN & CHEN, Proc Natl Acad Sci USA, 2013).

Les protéines FIDG appartiennent à la famille des AAA-ATPases (ATP-ases Associated with various Activities), et plus particulièrement au sous-groupe 7 (BEYER, Protein Sci, 6, 2043-58, 1997 ; FRICKEY & LUPAS, J Struct Biol, 146, 2-10, 2004). Ce sous-groupe comprend également les protéines KATANIN et SPASTIN qui sont, tout comme FIDGETIN, décrites comme des enzymes de sectionnement des microtubules. Elles ont été impliquées dans la régulation du nombre et de la taille de microtubules chez des nombreuses espèces : *C. elegans* (YAKUSHIJI et al., FEBS Lett, 578, 191-7, 2004) ; *D. melanogaster* (ZHANG et al., J Cell Biol, 177, 231-42, 2007) ; *H. sapiens* (MUKHERJEE et al., Cell Cycle, 11, 2359-66, 2012); *A. thaliana,* (STOPPIN-MELLET et al., Biochem J, 365, 337-42, 2002).

Les protéines FIDG apparaissent conservées chez les animaux et les plantes, mais semblent absentes chez les levures. Chez les animaux, de une à trois protéines apparentées ont été identifiés à FIDGETIN, (notamment trois chez les mammifères) ; en revanche, un seul représentant de cette famille a été identifié dans le génome du nématode *C. elegans,* du xénope, ainsi que dans ceux de la plupart des plantes.

Qu'elles soient animales ou végétales, les protéines FIDG présentent deux domaines protéiques conservés : un domaine AAA-ATPase, avec un rôle putatif d'hydrolyse de l'ATP ; ainsi qu'un domaine VSP4, connu pour jouer un rôle important dans la liaison aux microtubules. Ces deux domaines sont respectivement répertoriés sous les références PF00004 et PF09336 dans la base de données PFAM (PUNTA et al., Nucleic Acids Res. Database Issue 40:D290-D301, 2012).

L'inhibition dans une plante d'une protéine FIDG selon la présente invention entraîne une augmentation de la fréquence des COs méiotiques chez ladite plante.

La séquence de la protéine FIDG d'*Arabidopsis thaliana* déterminée par les Inventeurs par séquençage de l'ADN complémentaire (cDNA) dérivé de l'ARN messager codant FIDG, est représentée dans la liste de séquences en annexe sous le numéro SEQ ID NO: 1. La séquence du gène *FIDG* correspondant est une séquence composite des deux gènes chevauchants répertoriés sur la base de données TAIR10 sous les références AT3G27120 et AT3G27130. L'ARN messager est composé de 13 exons, le départ de traduction étant celui du gène prédit comme AT3G27130 et le codon stop appartenant au gène prédit comme AT3G27120 (la protéine est codée sur le brin Crick). Un site d'épissage mal prédit est à l'origine de la mauvaise annotation de ce gène dans les bases de données.

Une recherche dans les bases de données de séquences a permis d'identifier des orthologues d'*AtFIDG* chez un large panel d'eucaryotes, et il très probable que cette protéine est conservée dans l'ensemble des plantes terrestres. A titre d'exemple non limitatifs d'orthologues d'*AtFIDG* on citera notamment:
- la protéine de *Brachypodium distachyon* dont la séquence polypeptidique est disponible dans la base de données Genbank sous le numéro d'accès XM_003576467 ;
- la protéine de *Carica papaya* dont la séquence polypeptidique est prédite dans la base de données PLAZA sous le numéro d'accès CP00171G00260 ;
- la protéine de Fragaria vesca dont la séquence polypeptidique prédite figure dans la base de données Phytozome sous le numéro d'accès mrna25885.1 et dans la base de données PLAZA sous le numéro d'accès FV6G37220 ;
- chez *Glycin max,* deux gènes issus d'une duplication spécifique à cette espèce existent ; leur séquence polypeptidique est disponible dans la base de données Phytozome sous les numéros d'accès Glyma19g18350.2 et Glyma05g14440.2, et dans la base de données PLAZA sous les numéros d'accès respectifs GM19G18350 et GM05G14440;
- la protéine de *Oryza sativa* dont la séquence polypeptidique est disponible dans la base de données GenBank sous les numéros d'accès ABA97741 ou EEC69225.1, de préférence EEC69225.1 ;
- la protéine de *Populus tricocarpa* dont la séquence polypeptidique est disponible dans la base de données GenBank sous le numéro d'accès POPTR_0001s33870 ;
- la protéine de *Ricinus communis* dont la séquence polypeptidique est disponible dans la base de données Genbank sous le numéro d'accès XM_002509479 ;
- la protéine de *Solanum lycopersicum* dont la séquence polypeptidique est disponible dans la base de données Genbank sous le numéro d'accès XM_004233540.1 ;
- la protéine de *Sorghum bicolor* dont la séquence polypeptidique est disponible dans la base de données Genbank sous le numéro d'accès XM_002442067.1 ;
- la protéine de *Theobroma cacao* dont la séquence polypeptidique est prédite dans la base de données Phytozome sous le numéro d'accès Thecc1EG017182t1 ou Thecc1EG017182t2 (de préférence Thecc1EG017182t2) et dans la base de données PLAZA sous le numéro d'accès TC04G003320 ;
- la protéine de *Vitis vinifera* dont la séquence polypeptidique est disponible dans la base de données GenBank sous le numéro d'accès CBI21358 ;
- la protéine de *Zea mays* dont la séquence polypeptidique est disponible dans la base de données GenBank sous le numéro d'accès DAA54951 ;
- la protéine de *Hordeum vulgare* dont la séquence polypeptidique est disponible dans la base de données Genbank sous le numéro d'accès BAK02801.1 ;
- la protéine de *Triticum urartu* dont la séquence polypeptidique est disponible dans la base de données Genbank sous le numéro d'accès EMS65393.1.

L'objet de la présente invention est défini par les revendications 1 à 6.

La présente invention a pour objet un procédé pour augmenter la fréquence des COs méiotiques chez une plante, caractérisé en ce qu'il comprend l'inhibition dans ladite plante, d'une protéine ci-après dénommée FIDG, ladite protéine ayant au moins 45% d'identité de séquence, ou au moins 60% de similarité de séquence avec la protéine AtFIDG de séquence SEQ ID NO: 1, lesdits pourcentages étant calculés à l'aide de l'algorithme Needle EMBOSS en utilisant les paramètres par défaut pour le calcul d'identité et la matrice BLOSUM62 pour le calcul de similarité, et contenant un domaine AAA-ATPase et un domaine VSP4, respectivement répertoriés sous les références PFAM PF00004 et PF09336.

Dans un mode de réalisation, l'invention a pour objet un procédé tel que défini précédemment, caractérisé en ce qu'il comprend en outre l'inhibition dans ladite plante, d'une protéine ci-après dénommée FANCM, ladite protéine ayant au moins 30% d'identité de séquence, ou au moins 45% de similarité de séquence avec la protéine AtFANCM de séquence SEQ ID NO: 2, et contenant un domaine hélicase DEXDc et un domaine hélicase HELICc, respectivement répertoriés sous les numéros d'accession cd00046 et cd00079.

Dans un mode de réalisation, l'invention a pour objet un procédé tel que défini précédemment, caractérisé en ce que l'inhibition de la protéine FIDG et le cas échéant de la protéine FANCM, est obtenue par mutagenèse du gène codant pour ladite protéine ou de son promoteur.

Dans un mode de réalisation, l'invention a pour objet un procédé tel que défini précédemment, caractérisé en ce que l'inhibition de la protéine FIDG et le cas échéant de la protéine FANCM, est obtenue par extinction du gène codant pour ladite protéine en exprimant dans ladite plante un ARN interférent ciblant ledit gène.

Dans un mode de réalisation, l'invention a pour objet une plante mutante caractérisée en ce qu'elle contient une mutation dans le gène *FIDG,* ladite mutation induisant l'inhibition de la protéine FIDG dans ladite plante.

Dans un mode de réalisation, l'invention a pour objet une plante mutante telle que définie précédemment, caractérisée en ce qu'elle contient en outre une mutation dans le gène *FANCM,* ladite mutation induisant l'inhibition de la protéine FANCM dans ladite plante.

La présente description a pour objet une méthode pour augmenter la fréquence des COs méiotiques chez une plante, caractérisée en ce qu'elle comprend l'inhibition dans ladite plante, d'une protéine ci-après dénommée FIDG, ladite protéine ayant au moins 35%, et par ordre de préférence croissante, au moins 40, 45, 50, 55, 60, 65, 70, 75, 80, 85, 90, 95 ou 98% d'identité de séquence, ou au moins 50%, et par ordre de préférence croissante, au moins 50,55, 60, 65, 70, 75, 80, 85, 90, 95 ou 98% de similarité de séquence avec la protéine AtFIDG de séquence SEQ ID NO: 1, et contenant un domaine AAA-ATPase (PF00004) et un domaine VSP4 (PF09336).

De préférence, ladite protéine a au moins 70%, et par ordre de préférence croissante, au moins 75, 80, 85, 90, 95 ou 98% d'identité de séquence, ou au moins 80%, et par ordre de préférence croissante, au moins 85, 90, 95 ou 98% de similarité de séquence avec l'un quelconque des orthologues d'*AtFIDG* dont la liste est indiquée ci-dessus.

Selon un mode de mise en oeuvre préféré de la description, le domaine AAA-ATPase de ladite protéine FIDG a au moins 60%, et par ordre de préférence croissante, au moins 65, 70, 75, 80, 85, 90, 95 ou 98% d'identité de séquence, ou au moins 70%, et par ordre de préférence croissante, au moins 75, 80, 85, 90, 95 ou 98% de similarité de séquence avec le domaine AAA-ATPase de la protéine AtFIDG (acides aminés 431-561 de la SEQ ID NO: 1).

Selon un autre mode de mise en oeuvre préféré de la description, le domaine VSP4 de ladite protéine FIDG a au moins 60%, et par ordre de préférence croissante, au moins 65, 70, 75, 80, 85, 90, 95 ou 98% d'identité de séquence, ou au moins 70%, et par ordre de préférence croissante, au moins 75, 80, 85, 90, 95 ou 98% de similarité de séquence avec le domaine VSP4 de la protéine AtFIDG (acides aminés 623-672 de la SEQ ID NO: 1).

Avantageusement, ladite protéine FIDG contient en outre un domaine de liaison à RAD51, ayant au moins 50%, et par ordre de préférence croissante, au moins 55, 60, 65, 70, 75, 80, 85, 90, 95 ou 98% d'identité de séquence, ou au moins 70%, et par ordre de préférence croissante, au moins 75, 80, 85, 90, 95 ou 98% de similarité de séquence avec les acides aminés 268-346 de la SEQ ID NO: 1.

Sauf précisions contraire, les valeurs d'identité et de similarité de séquence indiquées ici sont calculées sur la totalité de la longueur des séquences comparées, en utilisant l'algorithme d'alignement global de Needleman-Wunsch (programme Needle EMBOSS avec les paramètres par défaut). Les calculs de similarité sont effectués en utilisant la matrice BLOSUM62.

L'inhibition peut notamment être obtenue par mutagenèse du gène *FIDG.* Par exemple, une mutation dans la séquence codante peut induire, selon la nature de la mutation, l'expression d'une protéine inactive, ou d'une protéine à activité réduite; une mutation au niveau d'un site d'épissage peut également altérer ou abolir la fonction de la protéine ; une mutation dans la séquence promotrice peut induire l'absence d'expression de ladite protéine, ou la diminution de son expression.

La mutagenèse peut être effectuée par exemple par la suppression de tout ou partie de la séquence codante ou du promoteur de *FIDG,* ou par insertion d'une séquence exogène, par exemple un transposon ou un ADN-T, au sein de ladite séquence codante ou dudit promoteur. Elle peut également être effectuée en induisant des mutations ponctuelles, par exemple par mutagénèse EMS, par radiations, ou par mutagénèse dirigée, par exemple à l'aide de nucléases TALENs (Transcription Activator-Like Effector Nucleases) ou de nucléases à doigt de zinc (CHRISTIAN et al., Genetics, 186, 757-61, 2010; CURTIN et al., Plant Gen., 5, 42-50, 2012).

Les allèles mutés peuvent être détectés par exemple par PCR, en utilisant des amorces spécifiques du gène *FIDG.*

Différentes méthodes de mutagénèse et de criblage à haut débit sont décrites dans l'art antérieur. A titre d'exemples, on peut citer des méthodes de type « TILLING » (Targeting Induced Local Lesions In Genome), décrit par McCALLUM et al., Plant Physiol., 123, 439-442, 2000). L'absence de fonctionnalité de FIDG chez les mutants peut être vérifiée sur la base des caractéristiques phénotypiques de leur descendance ; les plantes homozygotes pour une mutation inactivant le gène FIDG ont un taux de COs méiotiques supérieur à celui des plantes sauvages (ne portant pas la mutation dans le gène FIDG) dont elles sont issues. Généralement ce taux de COs méiotiques est supérieur d'au moins 50%, de préférence au moins 2 fois supérieur à celui des plantes sauvages dont elles sont issues.

Alternativement, l'inhibition de la protéine FIDG est obtenue par extinction (silencing) du gène *FIDG.* Différentes techniques d'extinction de gènes dans les plantes sont connues en elles-mêmes (pour revue voir par exemple : WATSON & GRIERSON, Transgenic Plants : Fundamentals and Applications (Hiatt, A, ed) New York : Marcel Dekker, 255-281, 1992 ; CHICAS & MACINO, EMBO reports, 21, 992-996, 2001). On peut citer l'inhibition antisens ou co-suppression, décrites par exemple dans les Brevets US°5190065 et US°5283323. Il est également possible d'utiliser des ribozymes ciblant l'ARNm de la protéine FIDG.

De préférence, l'extinction du gène *FIDG* est induite par interférence ARN ciblant ledit gène.

Un ARN interférent (ARNi) est un petit ARN qui peut éteindre un gène cible de manière séquence spécifique. Les ARN interférents comprennent en particulier les « petits ARN interférents » (siRNA) et les micro-ARN (miRNA).

Initialement, les constructions d'ADN pour exprimer des ARN interférents dans les plantes contenaient un fragment de 100 pb ou plus (généralement de 100 à 800 pb) de l'ADNc du gène ciblé, sous contrôle transcriptionnel d'un promoteur approprié. Actuellement, les constructions les plus largement utilisées sont celles qui peuvent produire un transcrit d'ARN en épingle à cheveux (hpRNA). Dans ces constructions, le fragment du gène cible est inversement répété, avec généralement une région d'espacement entre les répétitions (pour revue, cf. WATSON et al., FEBS Letters, 579, 5982-5987, 2005). On peut également utiliser des micro-ARN artificiels (amiRNAs) dirigés contre le gène *FIDG* (OSSOWSKI et al., The Plant Journal, 53, 674-690, 2008 ; SCHWAB et al., Methods Mol Biol., 592, 71-88, 2010 ; WEI et al., Funct Integr Genomics., 9, 499-511, 2009).

La présente description a pour objet des constructions d'ADN recombinant, et notamment des cassettes d'expression, produisant un ARNi permettant d'éteindre le gène *FIDG.*

Une cassette d'expression conforme à la description comprend une séquence d'ADN recombinant dont le transcrit est un ARNi, notamment un hpRNA ou un amiRNA, ciblant le gène *FIDG,* placée sous contrôle transcriptionnel d'un promoteur fonctionnel dans une cellule végétale.

Un large choix de promoteurs appropriés pour l'expression de gènes hétérologues dans des cellules végétales ou des plantes est disponible dans l'art.

Ces promoteurs peuvent être obtenus par exemple à partir de plantes, de virus de plantes, ou de bactéries comme *Agrobacterium.* Ils incluent des promoteurs constitutifs, à savoir des promoteurs qui sont actifs dans la plupart des tissus et cellules et dans la plupart des conditions environnementales, ainsi que des promoteurs tissu-spécifiques ou cellule spécifiques, qui sont actifs uniquement ou principalement dans certains tissus ou certains types de cellules, et des promoteurs inductibles qui sont activés par des stimuli physiques ou chimiques.

Des exemples de promoteurs constitutifs qui sont couramment utilisés dans les cellules végétales sont le promoteur 35S du virus de la mosaïque du chou-fleur (CaMV) décrit par KAY et al. (Science, 236, 4805, 1987), ou ses dérivés, le promoteur du virus de la mosaïque des nervures de manioc (CsVMV) décrit dans la Demande Internationale WO 97/48819, le promoteur de l'ubiquitine du maïs ou le promoteur « Actine-Intron-actine » du riz (McELROY et al., Mol. Gen. Genet., 231, 150-160, 1991 ; GenBank numéro d'accès S 44221).

Dans le cadre de la présente description, on pourra aussi utiliser un promoteur spécifique de la méiose (c'est-à-dire actif exclusivement ou préférentiellement dans les cellules en méiose). A titre d'exemple non limitatif on peut citer le promoteur DMC1 (KLIMYUK & JONES, Plant J., 11, 1-14, 1997).

Les cassettes d'expression de la description comprennent généralement un terminateur transcriptionnel, par exemple le terminateur 3'NOS de la nopaline synthase (DEPICKER et al., J. Mol. Appl. Genet., 1, 561-573, 1982), ou le terminateur 3'CaMV (FRANCK et al., Cell, 21, 285-294, 1980). Elles peuvent également comprendre d'autres éléments de régulation de la transcription tels que des amplificateurs.

Les constructions d'ADN recombinant conformes à la description englobent également des vecteurs recombinants contenant une cassette d'expression conforme à la description. Ces vecteurs recombinants peuvent également inclure un ou plusieurs gènes marqueurs, qui permettent la sélection des cellules ou plantes transformées.

Le choix du vecteur le plus approprié dépend notamment de l'hôte prévu et de la méthode envisagée pour la transformation de l'hôte en question. De nombreuses méthodes pour la transformation génétique de cellules végétales ou de plantes sont disponibles dans l'art, pour de nombreuses espèces végétales, dicotylédones ou monocotylédones. A titre d'exemples non-limitatifs, on peut citer la transformation médiée par virus, la transformation par microinjection, par électroporation, la transformation par microprojectiles, la transformation par *Agrobacterium,* etc.

La description a également pour objet une cellule-hôte comprenant une construction d'ADN recombinant conforme à la description. Ladite cellule-hôte peut être une cellule procaryote, par exemple une cellule d'*Agrobacterium,* ou une cellule eucaryote, par exemple une cellule végétale génétiquement transformés par une construction d'ADN de la description. La construction peut être exprimée transitoirement, elle peut aussi être incorporée dans un réplicon extrachromosomique stable, ou intégrée dans le chromosome.

La description fournit également un procédé pour produire une plante transgénique ayant un taux de COs méiotiques supérieur à celui de la plante sauvage dont elle est issue, caractérisé en ce qu'il comprend les étapes suivantes :
- la transformation d'une cellule végétale avec un vecteur contenant une cassette d'expression conforme à la description;
- la culture de ladite cellule transformée afin de régénérer une plante ayant dans son génome un transgène contenant ladite cassette d'expression.

La description englobe également des plantes génétiquement transformées par une construction d'ADN de la description. De préférence, lesdites plantes sont des plantes transgéniques, dans laquelle ladite construction est contenue dans un transgène intégré dans le génome de la plante, de sorte qu'il est transmis aux générations successives de plantes. L'expression de la construction d'ADN de la description résulte en une régulation négative de l'expression du gène *FIDG,* qui confère auxdites plantes transgéniques un taux de COs méiotiques supérieur à celui des plantes sauvages (ne contenant pas la construction d'ADN de la description) dont elles sont issues. Généralement ce taux de COs méiotiques est supérieur d'au moins 50%, de préférence au moins 2 fois supérieur à celui des plantes sauvages dont elles sont issues.

De manière particulièrement avantageuse, le taux de COs méiotiques peut encore être augmenté en combinant dans une même plante, l'inhibition de la protéine FIDG avec celle de la protéine FANCM. Dans ce cas, le taux de COs méiotiques est au moins 3 fois supérieur, de préférence au moins 5 fois supérieur à celui des plantes sauvages dont elles sont issues.

L'augmentation du taux de COs méiotiques par inhibition de la protéine FANCM est décrite dans la Demande PCT WO2013038376, dont le contenu est incorporé par référence à la présente description.

La protéine FANCM est définie comme une protéine ayant au moins 30% et par ordre de préférence croissante, au moins 35, 40, 45, 50, 55, 60, 65, 70, 75, 80, 85, 90, 95 ou 98% d'identité de séquence, ou au moins 45%, et par ordre de préférence croissante, au moins 50, 55, 60, 65, 70, 75, 80, 85, 90, 95 ou 98% de similarité de séquence avec la protéine AtFANCM d'*Arabidopsis thaliana* (GenBank : NP_001185141 ; UniProtKB : F4HYE4), et contenant un domaine hélicase DEXDc (cd00046) et un domaine hélicase HELICc (cd00079). De préférence, le domaine hélicase DEXDc a au moins 65%, et par ordre de préférence croissante, au moins 70,75, 80, 85, 90, 95 ou 98% d'identité de séquence, ou au moins 75%, et par ordre de préférence croissante, au moins 80,85, 90, 95 ou 98% de similarité de séquence avec le domaine DEXDc (acides aminés 129-272) de la protéine AtFANCM, et le domaine hélicase HELICc a au moins 60%, et par ordre de préférence croissante, au moins 65, 70, 75, 80, 85, 90, 95 ou 98% d'identité de séquence, ou au moins 70%, et par ordre de préférence croissante, au moins 75, 80, 85, 90, 95 ou 98% de similarité de séquence avec le domaine HELICc (acides aminés 445-570) de la protéine AtFANCM. La séquence polypeptidique de la protéine AtFANCM est représentée dans la liste de séquences en annexe sous le numéro SEQ ID NO: 2.

L'inhibition dans une plante d'une protéine FANCM telle que définie ci-dessus entraîne une augmentation de la fréquence des COs méiotiques chez ladite plante.

La présente invention a donc également pour objet :
- un procédé pour augmenter la fréquence des COs méiotiques chez une plante, comprenant l'inhibition dans ladite plante, de la protéine FIDG et de la protéine FANCM ;
- des plantes mutantes contenant dans le gène *FIDG* une mutation induisant l'inhibition de la protéine FIDG, et dans le gène *FANCM* une mutation induisant l'inhibition de la protéine FANCM ;

La présente invention peut s'appliquer notamment dans le domaine de l'amélioration des plantes, afin d'accélérer l'obtention de nouvelles variétés. Elle permet aussi de faciliter la recombinaison entre espèces apparentées, et donc l'introgression de caractères d'intérêt. Elle permet également d'accélérer l'établissement de cartes génétiques et les clonages positionnels.

La présente description s'applique à un large éventail de plantes d'intérêt agronomique monocotylédones ou dicotylédones. A titre d'exemples non-limitatifs, on peut citer le colza, le tournesol, la pomme de terre, le maïs, le blé, l'orge, le seigle, le sorgo, le riz, le soja, le haricot, la carotte, la tomate, la courgette, le poivron, l'aubergine, le navet, l'oignon, le pois, le concombre, le poireau, l'artichaut, la betterave, le choux, le chou-fleur, les salades, l'endive, le melon, la pastèque, le fraisier, le pommier, le poirier, le prunier, le peuplier, la vigne, le coton, la rose, la tulipe, etc.

La présente description sera mieux comprise à l'aide du complément de description qui va suivre, qui se réfère à des exemples non-limitatifs illustrant les effets de mutations du gène AtFIDG sur la recombinaison méiotique et le taux de Cos, ainsi qu'aux figures annexées :
Figure 1 : Graphique représentant le nombre de bivalents en métaphase I des suppresseurs (mutants *zmm-*/*-*) comparé avec celui de plantes sauvages et de plantes *zmm* homozygotes non-mutées à l'EMS. En abscisse, phénotype des plantes testées ; en ordonnée nombre de bivalents (en noir) et de paires univalents (en gris clair) par méiose.
Figure 2 : Graphique représentant les fréquences de recombinaison mesurées dans le double mutant *fidg-1 fancm-1.*

### Légende :

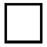

*: Sauvage* 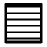
*: fidg-1* 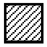
*: fancm* 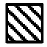
*: fidg-1 fancm-1*
Axe des abscisses : intervalles testés, selon la nomenclature de BERCHOWITZ & COPENHAVER (Nat. Protoc., 3, 41-50, 2008). I2a et I2b sont deux intervalles adjacents sur le chromosome 2 ; I3b et I3c sur le chromosome 3 ; I5c et I5d sur le chromosome 5. Axe des ordonnées : distance génétique (en cM).

### EXEMPLE 1 : OBTENTION DE MUTANTS SUPPRESSEURS DE MUTATIONS DE GENES ZMM

Des graines d'*Arabidopsis thaliana* homozygotes pour l'insertion d'ADN-T dans At*ZIP4*, *SHOC1, AtHEI10, AtMSH5 ou AtMSH4* (*Atzip4-*/*-, shoc1-*/*-, Athei10-*/*-, Atmsh5-*/*- ou Atmsh4-*/*-*) ont été mutées par EMS (éthylméthane sulfonate). Les plantes issues des graines mutées (population M1, hétérozygotes pour les mutations induites par l'EMS, et homozygotes pour la mutation du gène *ZMM*) ont un phénotype identique, résultant de l'inactivation du gène *ZMM,* qui se traduit par une diminution forte de la fréquence de CO, qui mène à une diminution forte du nombre de bivalents, et une baisse très importante de la fertilité (plantes « semi stériles »), résultant en la formation de siliques courtes qui se différencient facilement de celles des plantes sauvages. Les plantes M1 ont été auto-pollinisées, pour produire une population de descendants (population M2) contenant potentiellement des plantes homozygotes pour les mutations induites par l'EMS. Des plantes de la population M2 ayant des siliques plus longues que celles des plantes *zmm* homozygotes de la génération M1 ont été sélectionnées et génotypées afin de vérifier leur statut homozygote pour l'insertion d'ADN-T dans le gène *ZMM* concerné. Ce sont des suppresseurs. Le nombre de bivalents en métaphase I de ces suppresseurs a été comparé avec celui de plantes sauvages et de plantes *zmm* homozygotes non-mutées à l'EMS.

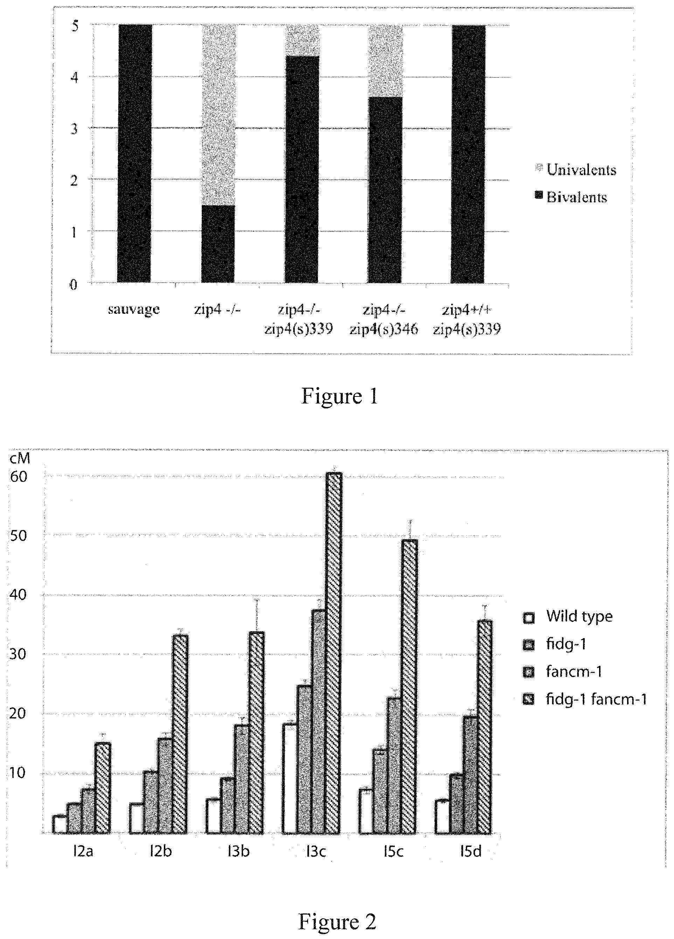

Les résultats sont illustrés par la Figure 1.

Alors que les plantes sauvages montrent systématiquement 5 bivalents par méiose, les mutants *zmm-*/*-,* notamment *Atzip4-*/*-,* montrent une forte diminution du nombre de bivalents (et donc l'apparition d'univalents). Les mutations dans *FIDGETIN* en fond *Atzip4-*/*-* (suppresseurs 339 et 346) permettent de restaurer partiellement la formation de bivalents. Les simples mutants *fidg* présentent, comme le sauvage, 5 bivalents.

Dans les plantes *zmm*/*SUPPRESSEUR,* on observe une mauvaise ségrégation des chromosomes qui résulte d'une diminution du nombre de bivalents, induite par la mutation *zmm,* et qui conduit à des gamètes déséquilibrés, non-viables. Au contraire, dans les plantes *zmm*/*suppresseur,* le nombre de bivalents est plus élevé, assurant une fertilité en partie restaurée. Les simple mutants *ZMM*/*suppresseur* se comportent comme les plantes sauvages, on observe une ségrégation normale des chromosomes, conduisant à la formation de gamètes équilibrés.

Les plantes *zmm*/*suppressor* et *ZMM*/*suppressor* ne présentent par ailleurs pas de différences somatiques phénotypiques visibles avec les plantes sauvages. Les mutations induites par l'EMS sont récessives. En effet, elles ne se traduisent pas par un phénotype détectable à l'état hétérozygote dans la population de mutants M1, et les plantes résultant du rétrocroisement des mutants *zmm*/*suppressor* avec les mutants *zmm* initiaux dont ils sont respectivement issus, ont un phénotype qui ne diffère pas de celui des mutants initiaux. Par ailleurs, la ségrégation des phénotypes « fertile » et « semi-stérile » chez les descendants issus de l'auto-pollinisation des plantes résultant du rétrocroisement des mutants *zmm*/*suppressor* avec les mutants *zmm* initiaux dont ils sont respectivement issus indique qu'un seul locus est concerné par la mutation.

### EXEMPLE 2 : LOCALISATION DANS LE GENE FIDGETIN

Parmi les mutants EMS décrits à l'Exemple 1 ci-dessus, deux lignées de mutants suppresseurs de la mutation du gène *ZIP4* (Figure 1), trois lignées de mutants suppresseurs de la mutation du gène *SHOC1,* trois lignées de mutants suppresseurs de la mutation du gène At*MSH5,* trois lignées de mutants suppresseurs de la mutation du gène *AtHEI10,* ainsi que une lignée de mutant suppresseurs de la mutation du gène *AtMSH4* ont été isolées. Ces lignées sont respectivement dénommées : *zip4(s)339, zip4(s)346, msh5(s)647, msh5(s)5, msh5(s)652, shoc1(s)101, shoc1(s)123, shoc1(s)161, hei10(s)141, hei10(s)208, hei10(s)235, msh4(s)80.*

Les mutations correspondantes ont toutes été localisées dans la séquence génomique d'*AtFIDGETIN.* Des tests de complémentation ont confirmé que ces mutations étaient bien responsables du phénotype observé.

Le Tableau I ci-dessous indique la nature et la position des mutations identifiées.

**Tableau I**

| Allèles de *FIDG* | | Changement dans la séquence nucléotidique (TAIR 10) | Changement dans la séquence protéique |
|---|---|---|---|
| *zip4(s)339* | *fidg-2* | Chr3 10 001 129: C>T | Site d'épissage modifié |
| *zip4(s)346* | *fidg-1* | Chr3 10 001 783: délétion 1pb | Décalage de phase, STOP |
| *msh5(s)647* | *fidg-3* | Chr3 10 000 278: C>T | STOP |
| *msh5 (s)5* | *fidg-4* | Chr3 10 004 090: C>T | STOP |
| *msh5(s)652* | *fidg-5* | Chr3 10 001 772: C>T | Changement d'acide aminé : E>K |
| *shoc1(s)101* | *fidg-6* | Chr3 10 001 017: G>A | Changement d'acide aminé: L>F |
| *shoc1(s)123* | *fidg-7* | Chr3 10 001 812: C>T | Site d'épissage modifié |
| *shoc1(s)161* | *fidg-8* | Chr3 10 001 909: G>A | Changement d'acide aminé: R>K |
| *hei10(s)141* | *fidg-9* | Chr3 10 001 970: C>T | STOP |
| *hei10(s)208* | *fidg-10* | Chr3 10 000 328: C>T | Changement d'acide aminé: E>K |
| *hei10(s)235* | *fidg-11* | Chr3 10 001 422: C>T | Changement d'acide aminé: D>N |
| *msh4(s)80* | *fidg-12* | Chr3 10 000 840 : C>T | Changement d'acide aminé: L>F |

### EXEMPLE 3 : INFLUENCE DE L'INACTIVATION DU GENE FIDG SUR LA FREQUENCE DE RECOMBINAISON MEIOTIQUE

Les fréquences de recombinaison méiotique chez des plantes mutantes *fidg* et sauvages ont été comparées. La fréquence de recombinaison méiotique a été mesurée par analyse de tétrades à l'aide de marqueurs fluorescents, comme décrit par BERCHOWITZ & COPENHAVER (Nat. Protoc., 3, 41-50, 2008). La distance génétique a été mesurée sur 2 intervalles adjacents sur le chromosome 2 (I2a et I2b), 2 intervalles adjacents du chromosome 3 (I3b et I3c), ainsi que 2 intervalles adjacents sur le chromosome 5 (intervalles I5c et I5d). Les résultats sont illustrés par la Figure 2 et le tableau II.

La mutation du gène *FIDGETIN* augmente la recombinaison par rapport au sauvage de 72% en moyenne sur les 6 intervalles.

### EXEMPLE 4 : INFLUENCE DE L'INACTIVATION COMBINEE DES GENES FIDG ET FANCM SUR LA FREQUENCE DE RECOMBINAISON MEIOTIQUE

De la même façon que dans l'exemple 2, les fréquences de recombinaison ont été mesurées dans le double mutant *fidg-1 fancm-1.* Les résultats sont représentés dans la Figure 2 et dans le Tableau II.

Le Tableau II représente les tailles génétiques (en cM) des différents intervalles chez les différents mutants (les erreurs standard sont indiquées entre parenthèses ; ND : non déterminé).

**Tableau II**

| cM | sauvage | +/- | *fidg-1* | +/- | *fancm-1* | +/- | *fidg-1 fancm-1* | +/- |
|---|---|---|---|---|---|---|---|---|
| I2a | 2,86 | 0,26 | 4,91 | 0,29 | 7,41 | 0,73 | 15,10 | 1,68 |
| I2b | 4,82 | 0,20 | 10,33 | 0,52 | 15,82 | 0,99 | 33,17 | 1,09 |
| I3b | 5,62 | 0,36 | 9,20 | 0,37 | 18,07 | 1,40 | 33,75 | 5,46 |
| I3c | 18,30 | 0,73 | 24,81 | 0,96 | 37,44 | 1,83 | 60,60 | 2,69 |
| I5c | 7,35 | 0,53 | 14,08 | 0,75 | 22,72 | 1,55 | 49,32 | 3,36 |
| I5d | 5,54 | 0,37 | 9,80 | 0,53 | 19,63 | 1,33 | 35,70 | 2,61 |
| I2a | 2,8 | (0,2) | 4,9 | (0,3) | 9,0 | (0,7) | ND | |
| I2b | 4,8 | (0,3) | 9,4 | (0,5) | 17,1 | (1,0) | ND | |
| I3c | 5,8 | (0,5) | 9,7 | (0,7) | 15,4 | (1,1) | ND | |
| I3d | 19,7 | (1,1) | 24,2 | (1,2) | 34,5 | (2,0) | 35,7 | |
| I5c | 6,2 | (0,1) | 9,6 | (0,5) | 17,8 | (0,6) | 49,3 | (2,6) |
| I5d | 6,3 | (0,1) | 13,5 | (0,8) | 21,5 | (0,8) | ND | (3,4) |

Ces résultats montrent que l'inactivation conjointe de *FIDG* et *FANCM* (*fidg-1 fancm-1*) conduit à une augmentation de la recombinaison d'en moyenne 5,77 fois par rapport au sauvage (moyenne sur les 6 intervalles).

## Revendications

1. Procédé pour augmenter la fréquence des COs méiotiques chez une plante, **caractérisé en ce qu'**il comprend l'inhibition dans ladite plante, d'une protéine ci-après dénommée FIDG, ladite protéine ayant au moins 45% d'identité de séquence, ou au moins 60% de similarité de séquence avec la protéine AtFIDG de séquence SEQ ID NO: 1, lesdits pourcentages étant calculés à l'aide de l'algorithme Needle EMBOSS en utilisant les paramètres par défaut pour le calcul d'identité et la matrice BLOSUM62 pour le calcul de similarité, et contenant un domaine AAA-ATPase et un domaine VSP4, respectivement répertoriés sous les références PFAM PF00004 et PF09336.

2. Procédé selon la revendication 1, **caractérisé en ce qu'**il comprend en outre l'inhibition dans ladite plante, d'une protéine ci-après dénommée FANCM, ladite protéine ayant au moins 30% d'identité de séquence, ou au moins 45% de similarité de séquence avec la protéine AtFANCM de séquence SEQ ID NO: 2, et contenant un domaine hélicase DEXDc et un domaine hélicase HELICc, respectivement répertoriés sous les numéros d'accession cd00046 et cd00079.

3. Procédé selon une quelconque des revendications 1 ou 2, **caractérisé en ce que** l'inhibition de la protéine FIDG et le cas échéant de la protéine FANCM, est obtenue par mutagenèse du gène codant pour ladite protéine ou de son promoteur.

4. Procédé selon une quelconque des revendications 1 à 3, **caractérisé en ce que** l'inhibition de la protéine FIDG et le cas échéant de la protéine FANCM, est obtenue par extinction du gène codant pour ladite protéine en exprimant dans ladite plante un ARN interférent ciblant ledit gène.

5. Plante mutante **caractérisée en ce qu'**elle contient une mutation dans le gène *FIDG,* ladite mutation induisant l'inhibition de la protéine FIDG dans ladite plante, ladite plante n'étant pas obtenue exclusivement au moyen d'un procédé essentiellement biologique.

6. Plante mutante selon la revendication 5, **caractérisée en ce qu'**elle contient en outre une mutation dans le gène *FANCM,* ladite mutation induisant l'inhibition de la protéine FANCM dans ladite plante.

## Patentansprüche

1. Verfahren, um die Frequenz der meiotischen COs bei einer Pflanze zu erhöhen, **dadurch gekennzeichnet, dass** es das Hemmen eines nachstehend FIDG genannten Proteins in der Pflanze umfasst, wobei das Protein mindestens 45 % Sequenzidentität oder mindestens 60 % Sequenzähnlichkeit mit dem AtFIDG Protein aus Sequenz SEQ ID-NR.: 1 aufweist, wobei die Prozentsätze mithilfe des Needle-EMBOSS-Algorithmus unter Verwendung der Standardparameter für die Identitätsberechnung, und der BLOSUM62-Matrix für die Ähnlichkeitsberechnung berechnet werden, und eine AAA-ATPase-Domäne und eine VSP4-Domäne enthält, die jeweils unter den Referenzen PFAM PF00004 und PF09336 gelistet sind.

2. Verfahren nach Anspruch 1, **dadurch gekennzeichnet, dass** es weiter das Hemmen eines nachstehend FANCM genannten Proteins in der Pflanze umfasst, wobei das Protein mindestens 30 % Sequenzidentität oder mindestens 45 % Sequenzähnlichkeit mit dem AtFANCM Protein aus Sequenz SEQ ID-NR.: 2 aufweist, und eine DEXDc-Helikasedomäne und eine HELICc-Helikasedomäne enthält, die jeweils unter den Zugangsnummern cd00046 und cd00079 gelistet sind.

3. Verfahren nach einem der Ansprüche 1 oder 2, **dadurch gekennzeichnet, dass** das Hemmen des FIDG Proteins und gegebenenfalls des FANCM Proteins durch Mutagenese des codierenden Gens für das Protein oder seinen Promotor erhalten wird.

4. Verfahren nach einem der Ansprüche 1 bis 3, **dadurch gekennzeichnet, dass** das Hemmen des FIDG Proteins und gegebenenfalls des FANCM Proteins durch Ausschalten des codierenden Gens für das Protein unter Exprimieren eines auf das Gen abzielenden RNA-Interferenten in der Pflanze erhalten wird.

5. Mutierte Pflanze, **dadurch gekennzeichnet, dass** sie eine Mutation im *FIDG* Gen enthält, wobei die Mutation das Hemmen des FIDG Proteins in der Pflanze induziert, wobei die Pflanze nicht ausschließlich mittels eines im Wesentlichen biologischen Verfahrens erhalten wird.

6. Mutierte Pflanze nach Anspruch 5, **dadurch gekennzeichnet, dass** sie weiter eine Mutation im *FANCM* Gen enthält, wobei die Mutation das Hemmen des FANCM Proteins in der Pflanze induziert.

## Claims

1. Method for increasing the frequency of meiotic CO in a plant, **characterised in that** it comprises the inhibition in said plant, of a protein hereinafter called FIDG, said protein having at least 45% sequence identity, or at least 60% sequence similarity with the protein AtFIDG of sequence SEQ ID NO: 1, said percentages being calculated with the EMBOSS needle algorithm using the default parameters for the identity calculation and the BLOSUM62 matrix for the similarity calculation, and containing an AAA-ATPase domain and a VSP4 domain, respectively listed under references PFAM PF00004 and PF09336.

2. Method according to claim 1, **characterised in that** it further comprises the inhibition in said plant of a protein hereinafter called FANCM, said protein having at least 30% sequence identity, or at least 45% sequence similarity with the protein AtFANCM of sequence SEQ ID NO: 2, and containing a helicase DEXDc domain and a helicase HELICc domain, respectively listed under accession numbers cd00046 and cd00079.

3. Method according to any one of the claims 1 or 2, **characterised in that** the inhibition of the FIDG protein and, where appropriate of the FANCM protein, is achieved by mutagenesis of the gene coding for said protein or its promoter.

4. Method according to any one of the claims 1 to 3, **characterised in that** the inhibition of the FIDG protein and, where appropriate of the FANCM protein, is achieved by silencing of the gene coding for said protein by expressing in said plant an interfering RNA targeting said gene.

5. Mutant plant **characterised in that** it contains a mutation in the *FIDG* gene, said mutation causing the inhibition of the FIDG protein in said plant, said plant not being exclusively obtained by means of an essentially biological process.

6. Mutant plant according to claim 5, **characterised in that** it further contains a mutation in the *FANCM* gene, said mutation inducing the inhibition of the FANCM protein in said plant.
